# EUROPEAN PATENT SPECIFICATION

(11) **EP 1 717 582 B1**
(45) Date of publication and mention of the grant of the patent: **15.06.2011**
(21) Application number: 05710685.8
(22) Date of filing: 18.02.2005
(51) Int. Cl.: G01N 33/52

(54) **MULTILAYER ANALYSIS ELEMENT**
MEHRSCHICHTIGES ANALYSEELEMENT
ELEMENT ANALYTIQUE MULTICOUCHE

(30) Priority: 20.02.2004 JP 2004044391
(43) Date of publication of application: 02.11.2006
(73) Proprietor: FUJIFILM Corporation, Minato-ku Tokyo (JP)
(72) Inventor: Nakamura, Kentaro, Asaka-shi, Saitama 3518585 (JP); Kawasaki, Kazuya, Asaka-shi, Saitama 3518585 (JP); Sakaino, Yoshiki, Asaka-shi, Saitama 3518585 (JP); Ito, Toshihisa, Asaka-shi, Saitama 3518585 (JP); Seshimoto, Osamu, Asaka-shi, Saitama 3518585 (JP)
(74) Representative: Albrecht, Thomas
(86) International application number: PCT/JP2005/003085
(87) International publication number: WO 2005/080979

(56) References cited:
- EP-A- 0 382 207
- EP-A- 0 621 478
- JP-A- 11 194 126
- JP-A- 2000 074 907
- JP-A- 2000 180 443
- JP-A- 2002 350 437
- US-A- 3 992 158

## Description

### Technical Field

The present invention relates to a multilayer analysis element used for clinical examinations, food examinations and environmental analysis or the like.

### Background Art

In the fields of clinical examination, food examination and environmental examination, there is a growing demand for processing a specimen quickly and easily, and analysis elements are generally employed to meet such needs. In an analysis element, the spreading layer that is used for the reception, spreading and diffusion of blood has been typically formed by a fibrous porous material, as described in JP Patent Publication (Kokai) Nos. 55-164356 A (1980), 57-66359 A (1982), and 60-222769 A (1985), for example.

The fibrous porous material has a high spreading rate upon spotting of a liquid sample and is easy to handle during manufacture. It is also compatible with viscous samples, such as whole blood, and is therefore widely used.

In the relevant fields, increasingly higher measurement accuracies (reproducibility) are being required, and several inconveniences have been identified in the fibrous porous material (fabric spreading layer). One of the inconveniencies relates to the problem of lot variations in the fabric. Normally, the fabric spreading layer is available in woven material and knitted material, and lot-to-lot and intra-lot differences in the manner of weaving or knitting have been identified. Specifically, the variations involve the number of stitches and weight per unit area, and thickness, for example. There are also lot-to-lot and intra-lot differences in the hydrophilicity of the fabric depending on the degree of washing in the material-washing step in an intermediate process. Furthermore, as the fabric spreading layer is not smooth, the spreading layer must inevitably be wedged into the lower layer if a sufficient bonding force is to be ensured by the laminating method during manufacture. As a result, the lower layer is disturbed and is not suitable for measurement requiring high accuracy. When adhering the fabric to the lower layer, the fabric, by its structural nature, tends to be elongated, such that the gap volume tends to be easily varied. Consequently, the spread area easily changes upon spotting of liquid sample, thereby producing the intra-lot difference and preventing a high-accuracy measurement. In recent years, there is also a growing need for performing a measurement with smaller amounts of liquid sample. In cases involving a fabric spreading layer, as the amount of liquid sample decreases, variations in the amount of reflected light become pronounced due to the influence of the stitches, such that it becomes impossible to perform a high-accuracy measurement.

As a means for addressing the above-mentioned drawbacks regarding a fabric spreading layer, an analysis element wherein a non-fibrous porous film is used for a spreading layer has been examined. The non-fibrous porous film is formed by coating, so that the accuracy of film formation is very high and the difference between lots and difference within a lot are also sufficiently small. Furthermore, the non-fibrous porous film is smoother and has larger specific surface area than the fabric spreading layer. Hence, the non-fibrous porous film can adhere to a lower layer with a desired adhesive strength without strongly breaking into the lower layer. Moreover, the non-fibrous porous film has no network such as is typical in the case of a fabric spreading layer, and it has high degree of whiteness. Hence, even with a smaller analysis element area, variations in the amount of reflected light are stable, and photometry with high accuracy is possible with the use of the non-fibrous porous film. From such viewpoints, JP Patent Publication (Kokoku) No. 53-21677 B (1978) and JP Patent Publication (Kokoku) No. 1-33782 B (1989) disclose spreading layers comprising non-fibrous porous films that comprise cellulose esters. However, these films have insufficient strength and thus may be problematic in terms of handling upon production. Thus, it is difficult to stably produce these films.

### Disclosure of the Invention

It is an object of the invention to solve the aforementioned problems of the prior art. Namely, it is an object of the invention to provide a multilayer analysis element with small intra-lot and lot-to-lot differences that has high measurement accuracy and which is small in size. Another object of the present invention is to provide a multilayer analysis element which can be stably produced.

As a result of intensive studies to solve the above objects, the present inventors have discovered that the above objects can be solved by the use of a non-fibrous porous film having a bending rupture strength of 20 gram-weight or more and a tensile percentage of 2% or less when the film is stretched with a tensile force of 50 gram-weight as a spreading layer. Thus, the present invention has been completed.

Specifically, the present invention provides a multilayer analysis element for liquid sample analysis wherein at least one functional layer and at least one porous liquid sample spreading layer of non-fibrous porous film are integrally laminated in this order on one side of a water-impermeable planar support, and the non-fibrous porous film has a bending rupture strength of 20 gram-weight or more and a tensile percentage of 2% or less when the film is stretched with a tensile force of 50 gram-weight.

Preferably, the non-fibrous porous film comprises a porous film of an organic polymer.

Preferably, the porous film of an organic polymer is an asymmetric porous film with an asymmetry ratio of 2.0 or more, or the porous film of an organic polymer is a symmetric porous film with an asymmetry ratio of less than 2.0.

Preferably, the porous film of an organic polymer is 6, 6-nylon; 6-nylon; acrylate copolymer; polyacrylate; polyacrylonitrile; polyacrylonitrile copolymer; polyamide, polyimide; polyamide-imide; polyurethane; polyether sulfone; polysulfone; a mixture of polyether sulfone and polysulfone; polyester; polyester carbonate; polyethylene; polyethylene chlorotrifluoroethylene copolymer; polyethylene tetrafluoroethylene copolymer; polyvinyl chloride; polyolefin; polycarbonate; polytetrafluoroethylene; polyvinylidene difluoride; polyphenylene sulfide; polyphenylene oxide; polyfluorocarbonate; polypropylene; polybenzoimidazole; polymethyl methacrylate; styrene-acrylonitrile copolymer; styrene-butadiene copolymer; a saponified product of ethylenevinyl acetate copolymer; polyvinyl alcohol; or a mixture thereof.

Further preferably, the porous film of an organic polymer is 6, 6-nylon; 6-nylon; polyether sulfone; polysulfone; a mixture of polyether sulfone and polysulfone; polyethylene; polypropylene; polyolefin; polyacrylonitrile; polyvinyl alcohol; polycarbonate; polyester carbonate; polyphenylene oxide; polyamide, polyimide; polyamide-imide; or a mixture thereof.

Particularly preferably, the porous film of an organic polymer is polyethersulfone, polysulfone, or a mixture thereof.

### BRIED DESCRIPTION OF THE DRAWINGS

Fig. 1 shows the optical arrangement of a photometry system used in the examples substance amount measuring device 100, specimen disposed portion 1, light source 2, light adjusting portion 3, wavelength adjusting portion 4, lenses 5a, 5b, 5c, area sensor 6 and computer 7 are shown.

Fig. 2 shows the relationship between the standard deviation (SD) of optical density and the photometry area.

### Best Mode for Carrying Out the Invention

The invention is hereafter described by way of embodiments thereof.

The multilayer analysis element of the invention for the analysis of a liquid sample is characterized in that at least one functional layer and at least one porous liquid sample spreading layer of non-fibrous porous film are integrally laminated in such order on one side of a water-impermeable planar support, and the non-fibrous porous film has a bending rupture strength of 20 gram-weight or more and a tensile percentage of 2% or less when the film is stretched with a tensile force of 50 gram-weight.

In the present invention, the non-fibrous porous film having a bending rupture strength of 20 gram-weight or more and a tensile percentage of 2% or less when the film is stretched with a tensile force of 50 gram-weight is used as a porous liquid sample spreading layer. With such characteristics, an effect of not causing a broken spreading layer (during the step of adhering a spreading layer to a lower layer) or the like upon production can be obtained. Furthermore, an effect of not causing any changes in void volume because of low tensile percentage can be obtained. Accordingly, a multilayer analysis element can be provided, which results in small difference between lots, small difference within a lot, and high measurement accuracy, can be miniaturized, and enables stable production.

In the present invention, the non-fibrous porous film to be used as a porous liquid sample spreading layer has a bending rupture strength of 20 gram-weight or more, preferably 30 gram-weight or more, and further preferably 50 gram-weight or more. The bending rupture strength in the present invention can be measured by bending a porous film (2 cm (width) x 5 cm (length)), placing a sash weight of predetermined weight on the thus formed loop portion, and then determining the weight that is added when the porous film is broken and destroyed.

In the present invention, the non-fibrous porous film to be used as a porous liquid sample spreading layer has a tensile percentage of 2% or less, preferably 1% or less, further preferably 0.5% or less, particularly preferably 0.1% or less, and most preferably 0.0% when the film is stretched with a tensile strength of 50 gram-weight. The tensile percentage when the film is stretched with a tensile force of 50 gram-weight in the present invention can be measured by cutting the non-fibrous porous film into a strip (2 cm x 8 cm), putting tapes on both ends of the film, making a hole on one end, fixing the end with no hole using a clip, hanging the strip, measuring the length of the strip with no load, hanging a sash weight of 50 gram-weight with the use of the hole, measuring the length of the strip to which the sash weight is applied, and then calculating the ratio of these lengths (specifically, the ratio of the length of the unloaded strip to the length of the strip to which the sash weight is applied).

As the water impermeable planar support, any conventional water impermeable support used in conventional analysis elements can be used. For example, it may be a film- or sheet-like transparent support made of a polymer, such as polyethylene terephthalate, bisphenol A polycarbonate, polystyrene, cellulosic ester (such as cellulose diacetate, cellulose triacetate, and cellulose acetate propionate, for example), with a thickness ranging from about 50 µm to about 1 mm, and preferably from about 80 µm to about 300 µm.

If necessary, the adhesion between the support and the functional layer provided thereon may be strengthened by providing an undercoat layer on the surface of the support. Alternatively, instead of the undercoat layer, the adhesion may be strengthened by subjecting the surface of the support to a physical or chemical activation process.

The multilayer analysis element of the invention comprises at least one porous liquid sample spreading layer of non-fibrous porous film. The porous liquid sample spreading layer is a layer having the function of spreading a component in an aqueous specimen in a planar fashion without substantially causing the component to be unevenly distributed, such that the component can be supplied to the functional layer at a substantially constant ratio per unit area.

The number of porous liquid sample spreading layers is not limited to one, and the layer may comprise a laminate of two or more layers of non-fibrous porous films adhered by an adhesive that is partially located. Physical activation treatment represented by glow discharge treatment or corona discharge treatment disclosed in Japanese Patent KOKAI 57-66359 may be conducted on at least one side of the aforementioned at least one porous liquid sample spreading layer of non-fibrous porous film. Alternatively, the porous liquid sample spreading layer may be subjected to immersing treatment with a surfactant (preferably, nonionic surfactant) disclosed in Japanese Patent KOKAI 55-164356, 57-66359, and so on, or hydrophilic treatment such as a immersing treatment with hydrophilic polymer, or a combination of two or more these treatment, and thereby the non-fibrous porous film is rendered hydrophilic. Thus, the adhesive force with the lower (the side which is near the support) functional layer can be strengthened, and the spreading area can be controlled. Further, a reagent for causing a desired detection reaction, a reagent for promoting the detection reaction, a variety of reagents for reducing or preventing an interfering or blocking reaction, or some of these reagents may be contained.

The thickness, pore size, porosity, water penetration rate or the like of the porous liquid sample spreading layer of non-fibrous porous film is not particularly limited. The thickness is in the range of 50 to 500 µm, preferably 50 to 350 µm, more preferably 80 to 200 µm. The average pore size is preferably 0.001 to 100 µm, more preferably 0.1 to 50 µm. The porosity is preferably 50 to 95% more preferably 60 to 90%. The water penetration rate is preferably 1 to 5000 ml, more preferably 5 to 2000 ml per minute per cm² of non-fibrous porous film, when water is transmitted at a pressure of 1 kg/cm² at 25°C.

The porous liquid sample spreading layer of the invention comprises a non-fibrous porous film. Preferably, the non-fibrous porous film is a porous film made of an organic polymer, which may be either symmetric or asymmetric. In the case of an asymmetric porous film, the asymmetry ratio is preferably 2.0 or more. In the case of a symmetric porous film, the asymmetry ratio is preferably not more than 2.0. The asymmetric porous film herein refers to a porous film having a larger mean diameter of pores on one surface than that on the other surface. The asymmetry ratio refers to the value obtained by dividing the larger mean pore diameter with the smaller mean pore diameter.

Preferable examples of the porous film made of an organic polymer include: 6, 6-nylon; 6-nylon; acrylate copolymer; polyacrylate; polyacrylonitrile; polyacrylonitrile copolymer; polyamide, polyimide; polyamide-imide; polyurethane; polyether sulfone; polysulfone; a mixture of polyether sulfone and polysulfone; polyester; polyester carbonate; polyethylene; polyethylene chlorotrifluoroethylene copolymer; polyethylene tetrafluoroethylene copolymer; polyvinyl chloride; polyolefin; polycarbonate; polytetrafluoroethylene; polyvinylidene difluoride; polyphenylene sulfide; polyphenylene oxide; polyfluorocarbonate; polypropylene; polybenzoimidazole; polymethyl methacrylate; styrene-acrylonitrile copolymer; styrene-butadiene copolymer; a saponified product of ethylenevinyl acetate copolymer; polyvinyl alcohol; and a mixture thereof. Of these, more preferable are: 6, 6-nylon; 6-nylon; polyether sulfone; polysulfone; a mixture of polyether sulfone and polysulfone; polyethylene; polypropylene; polyolefin; polyacrylonitrile; polyvinyl alcohol; polycarbonate; polyester carbonate; polyphenylene oxide; polyamide; polyimide; polyamide-imide; and a mixture thereof. Particularly, polyethersulfone, polysulfone, and a mixture thereof are preferable.

The non-fibrous porous film containing a reagent, which refers to a film containing a reagent, may be prepared by immersing a porous film in a solution of reagent and then drying it. In another method, the reagent-containing non-fibrous porous film may be prepared by applying a reagent solution to a porous film and then drying it. The means of preparing the film is not particularly limited.

The multilayer analysis element of the invention comprises at least one functional layer. The functional layer may consist of one or more layers.

Examples of the functional layer include: an adhesion layer for adhering a spreading layer and a functional layer; a water-absorbing layer for absorbing a liquid reagent; a mordant layer for preventing the diffusion of a dye produced by chemical reaction; a gas transmitting layer for selectively transmitting gas; an intermediate layer for suppressing or promoting the transport of substance between layers; an elimination layer for eliminating an endogenous substance; a light-shielding layer for enabling a stable measurement of reflected light; a color shielding layer for suppressing the influence of an endogenous dye; a separation layer for separating blood cells and plasma; a reagent layer containing a reagent that reacts with a target of analysis; and a coloring layer containing a coloring agent.

In an example of the invention, a hydrophilic polymer layer may be provided on the support as a functional layer via another layer as needed, such as an underlayer. The hydrophilic polymer layer may include: a nonporous, water-absorbing and water-permeable layer basically consisting only of a hydrophilic polymer; a reagent layer comprising a hydrophilic polymer as a binder and some or all of a coloring agent that is directly involved in a coloring reaction; and a detection layer containing a component (such as a dye mordant) that immobilizes the coloring agent in the hydrophilic polymer.

### (Reagent layer)

The reagent layer is a water-absorbing and water-permeable layer which contains a hydrophilic polymer binder in which at least some of a reagent composition that reacts with a detected component in an aqueous liquid to produce an optically detectable change is substantially uniformly dispersed. The reagent layer includes an indicator layer and a coloring layer.

A hydrophilic polymer that can be used as the binder in the reagent layer is generally a natural or synthetic hydrophilic polymer with a swelling rate ranging from about 150% to about 2000%, and preferably from about 250% to about 1500% at 30°C, upon water absorption. Examples of such a hydrophilic polymer include: a gelatin (such as acid-treated gelatin or deionized gelatin, for example) disclosed in JP Patent Publication (Kokai) No. 60-108753 A (1985); a gelatin derivative (such as phthalated gelatin or hydroxyacrylate graft gelatin, for example); agarose; pullulan; pullulan derivative; polyacrylamide; polyvinyl alcohol; and polyvinylpyrrolidone.

The reagent layer may be a layer appropriately cross-linked and cured using a crosslinking agent. Examples of the crosslinking agent include: for gelatin, known vinylsulfon crosslinking agent such as 1, 2-bis (vinylsulfonyl acetoamide)ethane and bis(vinylsulfonylmethyl)ether and aldehydes; and, for methallyl alcohol copolymer, aldehydes and epoxy compounds containing two glycidyl groups and the like.

The thickness of the reagent layer when dried is preferably in the range of about 1 µm to about 100 µm, and more preferably about 3 µm to about 30 µm. Preferably, the reagent layer is substantially transparent.

The reagent contained in the reagent layer or other layers in the multilayer analysis element of the invention may be appropriately selected depending on the tested substance.

For example, when analyzing ammonia (in cases where the tested substance is ammonia or ammonia producing substance), examples of a color ammonia indicator include: leuco dyes, such as leucocyanine dye, nitro-substituted leuco dye, and leucophthalein dye (see U.S. Patent No. Re. 30267 or JP Patent Publication (Kokoku) No. 58-19062 B (1983); pH indicators, such as bromophenol blue, bromocresol green, bromthymol blue, quinoline blue, and rosolic acid (see Encyclopaedia Chimica, Vol. 10, pp 63-65, published by Kyoritsu Shuppan K. K.); triarylmethane dye precursors; leucobenzylidene dyes (see JP Patent Publication (Kokai) Nos. 55-379 A (1980) and 56-145273 A (1981)); diazonium salt and azo dye couplers; and base bleaching dyes. The content of the color ammonia indicator with respect to the weight of the binder is preferably in the range of about 1 to about 20% by weight.

The reagent that reacts with an ammonia producing substance as a tested substance to produce ammonia is preferably an enzyme or a reagent that contains an enzyme, and the enzyme suitable for analysis may be selected appropriately depending on the type of the ammonia producing substance as the tested substance. When an enzyme is used as the regent, the combination of the ammonia producing substance and the reagent is determined by the specificity of the enzyme. Examples of the combination of the ammonia producing substance and the enzyme as the reagent include: urea/urease; creatinine/creatinine deiminase; amino acid/amino-acid dehydrogenase; amino acid/amino-acid oxidase; amino acid/ammonia lyase; amine/amine oxidase; diamine/amine oxidase; glucose and phosphoamidate/phosphoamidate-hexose phosphotransferase; ADP/carbamate kinase and carbamoyl phosphate; acid amide/amide hydrolase; nucleobase/nucleobase deaminase; nucleoside/nucleoside deaminase; nucleotide/nucleotide deaminase; guanine/guanase. An alkaline buffer that can be used in the reagent layer during the analysis of ammonia may be a buffer with a pH of 7.0 to 12.0, and preferably 7.5 to 11.5.

In addition to the reagent that reacts with an ammonia producing substance to produce ammonia, an alkaline buffer, and a hydrophilic polymer binder with a film-forming capability, the reagent layer for the analysis of ammonia may contain a wetting agent, a binder crosslinking agent (curing agent), a stabilizing agent, a heavy-metal ion trapping agent (complexing agent) or the like, as needed. The heavy-metal ion trapping agent is used for masking heavy-metal ions that inhibit enzyme activity. Examples of the heavy-metal ion trapping agent include complexanes such as: EDTA-2Na; EDTA.4Na; nitrilotriacetic acid (NTA); and diethylenetriaminepentaacetic acid.

Examples of the glucoses-measuring reagent composition include glucose oxidase, peroxidase, 4-aminoantipyrine or derivatives thereof, and an improved Trinder's reagent composition including 1,7-dihydroxynaphthalene, as described in U.S. Patent No. 3,992,158, JP Patent Publication (Kokai) Nos. 54-26793 A (1979), 59-20853 A (1984), 59-46854 A (1984), and 59-54962 A (1984).

### (Light-shielding layer)

A light-shielding layer may be provided on top of the reagent layer as needed. The light-shielding layer is a water-transmitting or water-permeable layer comprising a small amount of hydrophilic polymer binder with a film-forming capability in which particles with light-absorbing or light-reflecting property (together referred to as "light-shielding property") are dispersed. The light-shielding layer blocks the color of the aqueous liquid supplied to the spreading layer (to be described later) by spotting, particularly the color red of hemoglobin in the case where the sample is whole blood, when measuring detectable changes (in color or in coloration, for example) that developed in the reagent layer by reflection photometry from the light-transmitting support side. In addition, the light-shielding layer also functions as a light-reflecting layer or a background layer.

Examples of the particle with light-reflecting property include: titanium dioxide particles (microcrystalline particles of rutile type, anatase type, or brookite type, with a particle diameter of about 0.1 µm to about 1.2 µm); barium sulfate particles; aluminum particles; and microflakes. Examples of the light-absorbing particles include: carbon black, gas black, and carbon microbeads, of which titanium dioxide particles and barium sulfate particles are preferable. Particularly, anatase-type titanium dioxide particles are preferable.

Examples of the hydrophilic polymer binder with a film-forming ability include hydrophilic polymers similar to the hydrophilic polymer used for the manufacture of the aforementioned reagent layer, as well as regenerated cellulose of weak hydrophilicity and cellulose acetate. Of these, gelatin, gelatin derivatives, and polyacrylamide are preferable. Gelatin or gelatin derivatives may be used in a mixture with a known curing agent (crosslinking agent).

The light-shielding layer may be provided by applying an aqueous dispersion of light-shielding particles and a hydrophilic polymer onto the reagent layer by a known method and then drying. Alternatively, instead of providing the light-shielding layer, a light-shielding particle may be contained in the aforementioned spreading layer.

### (Adhesive layer)

An adhesive layer may be provided on top of the reagent layer in order to adhere and stack the spreading layer, via a layer such as a light-shielding layer as needed.

The adhesive layer is preferably made of a hydrophilic polymer such that the adhesive layer is capable of adhering the spreading layer when moistened or swollen with water, so that the individual layers can be integrated. Examples of the hydrophilic polymer that can be used for the manufacture of the adhesive layer are hydrophilic polymers similar to those hydrophilic polymers used for the manufacture of the reagent layer. Of these, gelatin, gelatin derivatives, and polyacrylamide are preferable. The dried-film thickness of the adhesive layer is generally about 0.5 µm to about 20 µm, preferably about 1 µm to about 10 µm.

The adhesive layer may be provided on any other desired layer (in addition to the reagent layer) for improving the adhesion between other layers. The adhesive layer may be provided by applying an aqueous solution of a hydrophilic polymer and, as needed, a surface active agent or the like onto the support or the reagent layer by a known method, for example.

### (Water-absorbing layer)

The multilayer analysis element of the invention may be provided with a water-absorbing layer between the support and the reagent layer. The water-absorbing layer is a layer comprised mainly of a hydrophilic polymer that becomes swollen by absorbing water, and it can absorb water in the aqueous liquid sample that has reached or permeated the boundary of the water-absorbing layer. The water-absorbing layer functions to promote the permeation of blood plasma, which is the aqueous liquid component in the case where the sample is whole blood, to the reagent layer. The hydrophilic polymer used in the water-absorbing layer may be selected from those used in the aforementioned reagent layer. For the water-absorbing layer, gelatin, gelatin derivatives, polyacrylamide, and polyvinyl alcohol are generally preferable. Particularly, the aforementioned gelatin and deionized gelatin are preferable. Most particularly, the aforementioned gelatin used in the reagent layer is preferable. The thickness of the water-absorbing layer when dried is about 3 µm to about100 µm, preferably about 5 µm to about 30 µm. The amount of coating is about 3 g/m² to about 100 g/m², and preferably about 5 g/m² to about 30 g/m². The pH of the water-absorbing layer upon use (during the implementation of analysis operation) may be adjusted by adding a pH buffer or a known basic polymer or the like in the water-absorbing layer, as will be described later. The water-absorbing layer may further contain a known dye mordant or a polymer dye mordant, for example.

### (Detection layer)

The detection layer is generally a layer in which a dye or the like produced in the presence of a detected component is diffused and optically detectable through a support, and it may comprise a hydrophilic polymer. The detection layer may contain a dye mordant, such as a cationic polymer for an anionic dye, for example. The water-absorbing layer generally refers to a layer in which the dye produced in the presence of the detected component is not substantially diffused, and it is distinguished from the detection layer in this respect.

The reagent layer, water-absorbing layer, adhesive layer, and spreading layer or the like may each contain a surfactant, of which one example is a nonionic surfactant. Examples of nonionic surfactant include: p-octylphenoxypolyethoxyethanol, p-nonylphenoxypolyethoxyethanol, polyoxyethylene oleyl ether, polyoxyethylene sorbitan monolaurate, p-nonylphenoxypolyglycidol, and octyl glucoside. By adding the nonionic surfactant in the spreading layer, its function of spreading the aqueous liquid sample (metering function) can be improved. By adding the nonionic surfactant in the reagent layer or the water-absorbing layer, the water in the aqueous liquid sample can be facilitated to be substantially uniformly absorbed by the reagent layer or the water-absorbing layer during analysis operation, so that the contact of the liquid with the spreading layer can take place quickly and substantially uniformly.

In order to laminate a porous liquid sample spreading layer of non-fibrous porous film onto a functional layer such as a reagent layer, a water-absorbing layer or an adhesive layer, a method disclosed in JP Patent Publication (Kokai) Nos. 55-164356 A (1980), and 57-66359 A (1982) and the like. Namely, after a functional layer such as a reagent layer, a water-absorbing layer or an adhesive layer is coated and before such layer is dried, or after the functional layer is dried, an aqueous solution containing water and a surfactant, an aqueous solution containing a surfactant and a hydrophilic polymer, an organic solvent, or an aqueous solution containing an organic solvent or the like is provided substantially uniformly using wire bar coating machine, a coating machine with die or the like, and thus the functional layer is swelled or dissolved. Then, a porous liquid sample spreading layer of non-fibrous porous film is substantially uniformly laminated onto the swelled or dissolved functional layer while a weak pressure is applied so as to be integrated therewith. When the functional layer is swelled or dissolved, it may be warmed by radiation heat of infra-red radiation heater, or may be warmed by contacting the support with a heater.

The tested substance that can be analyzed by the multilayer analysis element of the invention is not particularly limited, and a particular component in an any liquid sample (including bodily fluids, such as whole blood, blood plasma, blood serum, lymph fluid, urine, saliva, cerebrospinal fluid, and vaginal fluid; drinking water, liquors, river water, and factory waste water) can be analyzed. For example, the multilayer analysis element can be used for the analysis of albumin (ALB), glucose, urea, bilirubin, cholesterol, proteins, enzymes (including blood enzymes such as LDH (lactate dehydrogenase), CPK (creatine kinase), ALT (alanine aminotransferase), AST (aspartate aminotransferase), and γGT (γ-glutamyltranspeptidase).

The multilayer analysis element of the invention can be prepared by known methods. Hemolysis reagent may be added in a reagent solution in advance for coating or impregnation. In another method, an aqueous solution, an organic solvent (ethanol or methanol, for example), or a solution of a water-organic solvent mixture of the hemolysis reagent, either alone or in combination with a surface active agent or a hydrophilic polymer for spread area control, may be applied onto the spreading layer for impregnation. The tested substance may be analyzed using this in accordance with a known method.

For example, the multilayer analysis element of the invention may be cut into small pieces of squares with each side measuring about 5 mm to about 30 mm, or circles of similar sizes. They can then be accommodated in a slide frame such as those described in JP Patent Publication (Kokoku) No. 57-283331 B (1982) (corresponding to U.S. Patent No. 4169751), JP Utility Model Publication (Kokai) No. 56-142454 U (1981) (corresponding to U.S. Patent No. 4387990), JP Patent Publication (Kokai) No. 57-63452 A (1982), JP Utility Model Publication (Kokai) No. 58-32350 U (1983), and JP Patent Publication (Kohyo) No. 58-501144 A (1983) (corresponding to WO083/00391), and the slide can then be used as a chemical analysis slide. This is preferable from the viewpoint of manufacture, packaging, shipping, storage, measurement operation, and so on. Depending on the purpose of use, the element may be stored in a cassette or a magazine in the form of an elongated tape. Alternatively, small pieces may be stored in a container with an opening, they may be affixed to or accommodated in an opening card, or the cut pieces may be used as is.

In the multilayer analysis element of the invention, about 2 µL to about 30 µL, and preferably 4 µL to 15 µL of an aqueous liquid sample is spotted on the porous liquid sample spreading layer. The thus spotted multilayer analysis element is then incubated at a certain temperature ranging from about 20°C to about 45°C, preferably from about 30°C to about 40°C, for 1 to 10 minutes. The coloration or change in color in the multilayer analysis element is measured from the support side by reflection photometry, and the amount of the tested substance in the specimen can be determined using a prepared analytical curve based on the principle of colorimetry.

A highly accurate quantitative analysis can be performed by a very simple procedure using a chemical analyzer such as those disclosed in JP Patent Publication (Kokai) Nos. 60-125543 A (1985), 60-220862 A (1985), 61-294367 A (1986), 58-161867 A (1983) (corresponding to U.S. Patent No. 4424191), for example. Depending on the purpose or the desired level of accuracy, the degree of coloration may be judged visually and a semiquantitative analysis may be performed.

Since the multilayer analysis element of the invention is stored in a dry state until the beginning of analysis, there is no need to prepare a reagent as required. Further, as the reagents are generally more stable in a dry state, the multilayer analysis element of the invention can be more simply and quickly utilized than the so-called wet methods, in which solutions of reagents must be prepared as required. The invention is also superior as an examination method whereby a highly accurate examination can be performed with small quantities of liquid sample.

The invention will be hereafter described in more detail by way of examples thereof. The invention is not limited by these examples.

### Examples

### Example 1: Comparison of disruptive strengths upon bending of spreading layer materials

A polysulfone film (SE-200: produced by Fuji Photo Film Co., Ltd., hereinafter referred to as PS film), a polyethersulfone film (Precision: produced by Pall Corporation, hereinafter referred to as PES film), and a cellulose acetate film (FM-300: produced by Fuji Photo Film Co., Ltd., hereinafter referred to as FM film) were each cut into the shape of a strip (2 cm x 5 cm). One end of each strip was superimposed on the other end to form a loop portion at the center. 50 g, 20 g, or 10 g of a sash weight was gently placed on the loop portion, and then the strip was allowed to stand for 2 to 3 seconds. Subsequently, the strip was bent into the opposite direction. The above sash weight was placed on the previously bent portion and then the strip was allowed to stand for 2 to 3 seconds. After the procedure, frequencies of breaking strips were confirmed. Table 1 shows the number of strips that had been broken when the test was conducted.

**Table 1. Evaluation results of bending test**

| Sash weight (gram-weight) | PS film | PES film | FM film |
|---|---|---|---|
| 50 | 0/5 strips | 0/5 strips | 5/5 strips |
| 20 | 0/5 strips | 0/5 strips | 5/5 strips |
| 10 | 0/5 strips | 0/5 strips | 3/5 strips |

It could be confirmed that whereas the FM film is broken 100% with a sash weight of 20 gram-weight or more, the PS film and the PES film are not broken. The above results also suggest that the stability of the PS film and the same of the PES film are very high when handled upon production.

### Example 2: Comparison of tensile percentages of spreading layer materials

A polysulfone film (SE-200: produced by Fuji Photo Film Co., Ltd., hereinafter referred to as PS film), a polyethersulfone film (precision: produced by Pall Corporation, hereinafter referred to as PES film), and tricot woven fabric (hereinafter referred to as woven fabric) produced through 36 gauge-knitting of polyethylene terephthalate spun yarn having a fineness of 50 deniers were each cut into a strip (2 cm × 8 cm). Tapes were put on both ends of the film and then a hole was made on one end. The end with no hole was fixed using a clip, thereby hanging the strip. The length of the strip with no load was measured. Subsequently, a sash weight of 50 gram-weight was hanged with the use of the hole and then the length of the strip to which the sash weight was applied was measured. The tensile percentages were calculated. Measurement was performed at 22°C and humidity of 36%. Table 2 shows tensile percentages which were determined in this experiment.

**Table 2. Tensile percentages when the strips were stretched with a tensile strength of 50 gram-weight**

| | PS film | PES film | Woven fabric |
|---|---|---|---|
| Length of the strip with no load (cm) | 4.98 | 4.93 | 5.05 |
| Length of the strip to which the sash weight was applied (cm) | 4.98 | 4.93 | 5.18 |
| Tensile percentage | 0.0% | 0.0% | 2.6% |

It could be confirmed that whereas the woven fabric was stretched under this experimental conditions, the PS film and the PES film were not stretched at all. As shown in the above results, the PS film and the PES film do not change upon production, so as to be able to achieve smaller difference within a lot and smaller difference between lots.

Example 3: Sensitivity and accuracy of multilayer analysis elements for Ca, ALB and ALT analyses using various spreading layer materials.

### (1) Method for producing a multilayer analysis element for Ca analysis using a polysulfone film as a spreading layer

A transparent, colorless and gelatin-subbed polyethylene terephthalate smooth film of 180 µm was coated with an aqueous solution (pH=5.6) having the following composition to result in the following coating amounts. The film was dried so as to prepare a water absorption layer.

| | |
|---|---|
| Gelatin | 11.7 g/m² |
| 1 N potassium hydroxide | 1.2 g/m² |
| Titanium oxide | 0.8 g/m² |
| Polyoxy(2-hydroxy)propylene nonylphenylether | 0.3 g/m² |

Next, an aqueous solution (pH=5.3) having the following composition was applied onto the above water absorption layer to result in the following coating amounts. The layer was dried, so as to prepare a reagent layer.

| | |
|---|---|
| Gelatin | 11.9 g/m² |
| 2-morpholinoethanesulfonic acid monohydrate | 6.4 g/m² |
| 1 N potassium hydroxide | 7.0 g/m² |
| CPA(III) | 0.4 g/m² |
| Polyoxy(2-hydroxy)propylene nonylphenylether | 0.4 g/m² |
| Di-iso-octylsodium sulfosuccinate | 0.1 g/m² |

Next, water was supplied in an amount of approximately 15 g/m² onto the whole surface of the above reagent layer, so as to impregnate the layer with water. A polysulfone film (SE-200: produced by Fuji Photo Film Co., Ltd., hereinafter referred to as PS film) was laminated on the layer using slight pressure. The laminated product was dried, so as to cause the film to adhere to the layer. As described above, a multilayer analysis element for Ca analysis was produced using the polysulfone film as a spreading layer.

The above multilayer analysis element was cut into a 12 mm×13 mm chip. The chip was set in a slide frame (disclosed in JP Patent Publication (Kokai) No. 57-63452 A (1982)), to produce slide 1 for Ca analysis.

### (2) Method for producing a multilayer analysis element for Ca analysis using a woven fabric as a spreading layer

A water absorption layer and a reagent layer were produced in a manner similar to that in (1) the method for producing a multilayer analysis element for Ca analysis using a polysulfone film as a spreading layer. Water was supplied in an amount of approximately 30 g/m² onto the whole surface of the reagent layer, so as to impregnate the layer with water. Tricot woven fabric (hereinafter referred to as woven fabric) produced through 36 gauge-knitting of polyethylene terephthalate spun yarn having a fineness of 50 deniers was laminated onto the layer using slight pressure. The laminated product was dried, so as to cause the woven fabric to adhere to the layer.

An ethanol solution having the following composition was applied onto the above woven fabric to result in the following coating amounts. The woven fabric was dried, so that a multilayer analysis element for Ca analysis was produced using the woven fabric as a spreading layer.

| | |
|---|---|
| Polyvinyl pyrrolidone | 3.3 g/m² |
| Polyoxyethylene (10)octylphenylether | 0.4 g/m² |

The above multilayer analysis element was cut into a 12 mm×13 mm chip. The chip was set in a slide frame (disclosed in JP Patent Publication (Kokai) No. 57-63452 A (1982)), to produce slide 2 for Ca analysis.

### (3) Sensitivity comparison and measurement accuracy comparison

Water and a control serum with a Ca concentration of 15.2 mg/dl were each spotted (10 µL each) on the above slides 1 and 2 for Ca analysis. Subsequently, each slide was subjected to incubation at 37°C. Reflection optical density at 625 nm was measured from the support side to determine each reflection optical density 4 minutes after spotting. Sensitivities (difference between reflection optical density measured when the control serum having a Ca concentration of 15.2 mg/dl had been spotted and reflection optical density measured when water had been spotted (dODr (15.2 mg/dl-0 mg/dl))) were compared. Furthermore, accuracies (CV(%)) were compared when the control sera having Ca concentrations of 6.1, 9.7, and 15.2 mg/dl, respectively, had been spotted (10 µL each). Table 3 shows the results of sensitivity comparison and Table 4 shows the results of accuracy comparison.

**Table 3. Results of sensitivity comparison**

| | Sensitivity <d0Dr(15.2mg/d1-0mg/d1)> | Sensitivity ratio |
|---|---|---|
| Dry slide 1 for Ca analysis | 0.350 | 1.13 |
| Dry slide 2 for Ca analysis | 0.310 | 1 |

**Table 4. Results of accuracy comparison (CV(%))**

| Ca concentration (mg/dl) | Dry slide 1 for Ca analysis | Dry slide 2 for Ca analysis |
|---|---|---|
| 6.1 | 1.2% | 1.5% |
| 9.7 | 0.7% | 0.9% |
| 15.2 | 0.7% | 1.0% |

The multilayer analysis element 1 for Ca analysis using the PS film as a spreading layer showed more improved sensitivity than that of the multilayer analysis element 2 for Ca analysis using the woven fabric as a spreading layer. Furthermore, measurement accuracy was successfully improved in the case of the multilayer analysis element 1 for Ca analysis.

### (4) Method for producing a multilayer analysis element for ALB analysis using a polysulfone film as a spreading layer

A transparent, colorless and gelatin-subbed polyethylene terephthalate smooth film of 180 µm was coated with an aqueous solution (pH=2.8) having the following composition to result in the following coating amounts. The film was dried so as to prepare a reagent layer.

| | |
|---|---|
| Acrylamide-N-vinyl pyrrolidone-methacryl alcohol copolymer | 49.6 g/m² |
| DL-malic acid | 4.8 g/m² |
| Bromocresol green | 0.7 g/m² |
| Glycerine | 4.8 g/m² |
| Polyoxy(2-hydroxy)propylene nonylphenylether | 1.5 g/m² |

Next, a water-ethanol (1:1) mixed solution was supplied in an amount of approximately 15 g/m² onto the whole surface of the above reagent layer, so as to impregnate the layer with the mixed solution. A polysulfone film (SE-200: produced by Fuji Photo Film Co., Ltd., hereinafter referred to as PS film) was laminated on the layer using slight pressure. The laminated product was dried, so as to cause the film to adhere to the layer.

As described above, a multilayer analysis element for ALB analysis was produced using the polysulfone film as a spreading layer.

The above multilayer analysis element was cut into a 12 mmx13 mm chip. The chip was set in a slide frame (disclosed in JP Patent Publication (Kokai) No. 57-63452 A (1982)), to produce slide 1 for ALB analysis.

### (5) Method for producing a multilayer analysis element for ALB analysis using a woven fabric as a spreading layer

A reagent layer was produced in a manner similar to that in (4) the method for producing a multilayer analysis element for ALB analysis using a polysulfone film as a spreading layer. A water-ethanol (1:1) mixed solution was supplied in an amount of approximately 30 g/m² onto the whole surface of the above reagent layer, so as to impregnate the layer with the mixed solution. Tricot woven fabric (hereinafter referred to as woven fabric) produced through 36 gauge-knitting of polyethylene terephthalate spun yarn having a fineness of 50 deniers was laminated onto the layer using slight pressure. The laminated product was dried, so as to cause the woven fabric to adhere to the layer.

An ethanol solution having the following composition was applied onto the above woven fabric to result in the following coating amounts. The woven fabric was dried, so that a multilayer analysis element for ALB analysis was produced using the woven fabric as a spreading layer.

| | |
|---|---|
| Polyvinyl pyrrolidone | 6.6 g/m² |
| DL-malic acid | 6.6 g/m² |
| Polyoxyethylene(7)oleylether | 3.3 g/m² |

The above multilayer analysis element was cut into a 12 mmx13 mm chip. The chip was set in a slide frame (disclosed in JP Patent Publication (Kokai) No. 57-63452 A (1982)), to produce slide 2 for ALB analysis.

### (6) Sensitivity comparison and measurement accuracy comparison

Water and a control serum with an ALB concentration of 6.0 g/dl were each spotted (10 µL each) on the above slides 1 and 2 for ALB analysis. Subsequently, each slide was subjected to incubation at 37°C. Reflection optical density at 625 nm was measured from the support side to measure each reflection optical density 4 minutes after spotting. Sensitivities (difference between reflection optical density measured when the control serum having an ALB concentration of 6.0 g/dl had been spotted and reflection optical density measured when water had been spotted (dODr (6.0 g/dl-0 g/dl))) were compared. Furthermore, accuracies (standard deviation<SD>g/dl) were compared when the control sera having ALB concentrations of 1.7, 3.9, and 6.0 g/dl, respectively, had been spotted (10 µL each). Table 5 shows the results of sensitivity comparison and Table 6 shows the results of accuracy comparison.

**Table 5. Results of sensitivity comparison**

| | Sensitivity <d0Dr(6.0g/d1-0g/d1)> | Sensitivity ratio |
|---|---|---|
| Dry slide 1 for ALB analysis | 0.766 | 1.08 |
| Dry slide 2 for ALB analysis | 0.707 | 1 |

**Table 6. Results of accuracy (standard deviation<SD>g/dl) comparison**

| ALB concentration (g/dl) | Dry slide 1 for ALB analysis | Dry slide 2 for ALB analysis |
|---|---|---|
| 1.7 | 0.04 | 0.03 |
| 3.9 | 0.04 | 0.08 |
| 6.0 | 0.01 | 0.10 |

The multilayer analysis element 1 for ALB analysis using the PS film as a spreading layer showed more improved sensitivity than that of the multilayer analysis element 2 for ALB analysis using the woven fabric as a spreading layer. Furthermore, measurement accuracy was successfully improved in the case of the multilayer analysis element 1 for ALB analysis.

### (7) Method for producing a multilayer analysis element for ALT analysis using a polysulfone film as a spreading layer

A transparent, colorless and gelatin-subbed polyethylene terephthalate smooth film of 180 µm was coated with an aqueous solution (pH=6.5) having the following composition to result in the following coating amounts. The film was dried so as to prepare a reagent layer.

| | |
|---|---|
| Gelatin | 18.5 g/m² |
| Polyoxy(2-hydroxy)propylene nonylphenylether | 0.7 g/m² |
| Peroxidase | 14.8 KU/m² |
| Flavin adenine dinucleotide disodium | 0.02 g/m² |
| Cocarboxylase | 0.09 g/m² |
| 2-(4-hydroxy-3,5-dimethoxyphenyl-4-[4-(dimethylamino)phenyl]-5-phenethylimidazole (leucodye) acetate | 0.28 g/m² |
| Pyruvate oxidase | 3.4 KU/m² |
| Bis[(vinylsulfonylmethylcarbonyl)amino] methane | 0.6 g/m² |

Next, water was supplied in an amount of approximately 15 g/m² onto the whole surface of the above reagent layer, so as to impregnate the layer with water. A polysulfone film (SE-200: produced by Fuji Photo Film Co., Ltd., hereinafter referred to as PS film) was laminated on the layer using slight pressure. The laminated product was dried, so as to cause the film to adhere to the layer.

An aqueous solution (pH=7.5) having the following composition was applied onto the above polysulfone film to result in the following coating amounts. The polysulfone film was dried, so that a multilayer analysis element for ALT analysis using the polysulfone film as a spreading layer was produced.

| | |
|---|---|
| Polyethylene glycol(10)octylphenylether | 0.2 g/m² |
| Polyethylene glycol(40)octylphenylether | 1.32 g/m² |
| Cellulose ether (Metlose 90SH100) | 0.83 g/m² |
| Tris(hydroxymethyl)aminomethane | 0.36 g/m² |
| Monopotassium phosphate | 0.43 g/m² |
| α-ketoglutaric acid disodium dihydrate | 0.38 g/m² |
| L-alanine | 2.6 g/m² |
| Magnesium chloride hexahydrate | 0.23 g/m² |
| Ascorbate oxidase | 4.5 KU/m² |

The above multilayer analysis element was cut into a 12 mmx13 mm chip. The chip was set in a slide frame (disclosed in JP Patent Publication (Kokai) No. 57-63452 A (1982)), to produce slide 1 for ALT analysis.

### (8) Method for producing a multilayer analysis element for ALT analysis using a woven fabric as a spreading layer

A reagent layer was produced in a manner similar to that in (7) the method for producing a multilayer analysis element for ALT analysis using a polysulfone film as a spreading layer. Water was supplied in an amount of approximately 30 g/m² onto the whole surface of the above reagent layer, so as to impregnate the layer with water. Broad fabric (hereinafter referred to as woven fabric) produced through knitting of polyethylene terephthalate spun yarn having a yarn count of 60 was laminated onto the layer using slight pressure. The laminated product was dried, so as to cause the woven fabric to adhere to the layer. Furthermore, an aqueous solution (that is the same as that used in (7) the method for producing the multilayer analysis element for ALT analysis using the polysulfone film as a spreading layer) was applied onto the fabric. The fabric was dried. Thus, a multilayer analysis element for ALT analysis was produced using the woven fabric as a spreading layer.

The above multilayer analysis element was cut into a 12 mm × 13 mm chip. The chip was set in a slide frame (disclosed in JP Patent Publication (Kokai) No. 57-63452 A (1982)), to produce slide 2 for ALT analysis.

### (9) Sensitivity comparison and measurement accuracy comparison

Water and a control serum with ALT activity of 841 U/L were each spotted (10 µL each) on the above slides 1 and 2 for ALT analysis. Subsequently, each slide was subjected to incubation at 37°C. Reflection optical density at 650 nm was measured from the support side to determine the amount of change from reflection optical density measured 3 minutes after spotting to the same measured 4 minutes after spotting. Sensitivities (difference between the amount of change in reflection optical density measured when the control serum having ALT activity of 841U/L had been spotted and the amount of change in reflection optical density measured when water had been spotted (ddODr(841U/L-0U/L))) were compared. Furthermore, accuracies (CV(%)) measured when the control serum having ALT activity of 388, 841 U/L had been spotted (10 µL) were also compared. Table 7 shows the results of sensitivity comparison and Table 8 shows the results of accuracy comparison.

**Table 7. Results of sensitivity comparison**

| | Sensitivity <ddODr(841 U/L-OU/L)> | Sensitivity ratio |
|---|---|---|
| Slide I for ALT analysis | 0.240 | 1.39 |
| Slide 2 for ALT analysis | 0.173 | 1 |

**Table 8. Results of accuracy (CV(%)) comparison**

| ALT activity (U/L) | Slide 1 for ALT analysis | Slide 2 for ALT analysis |
|---|---|---|
| 388 | 1.2% | 2.2% |
| 841 | 1.1% | 3.3% |

The multilayer analysis element 1 for ALT analysis using the PS film as a spreading layer showed more improved sensitivity than that of the multilayer analysis element 2 for ALT analysis using the woven fabric as a spreading layer. Furthermore, measurement accuracy was successfully improved in the case of the multilayer analysis element 1 for ALT analysis.

### Example 4: Comparison of nonuniformities in reflected light quantities measured when various spreading layer materials were used and comparison of dispersions measured with smaller photometric areas

### (1) Method for producing a multilayer analysis element for reflected light quantity measurement using a polysulfone film as a spreading layer

A transparent, colorless and gelatin-subbed polyethylene terephthalate smooth film of 180 µm was coated with an aqueous solution (pH=5.1) having the following composition to result in the following coating amounts. The film was dried so as to prepare a water absorption layer.

| | |
|---|---|
| Gelatin | 16.6 g/m² |
| Polyoxy(2-hydroxy)propylene nonylphenylether | 0.2 g/m² |

Next, water was supplied in an amount of approximately 15 g/m² onto the whole surface of the above reagent layer, so as to impregnate the layer with water. A polysulfone film (SE-200: produced by Fuji Photo Film Co., Ltd., hereinafter referred to as PS film) was laminated on the layer using slight pressure. The laminated product was dried, so as to cause the film to adhere to the layer. As described above, a multilayer analysis element for reflected light quantity measurement was produced using the polysulfone film as a spreading layer.

The above multilayer analysis element was cut into a 12 mm×13 mm chip. The chip was set in a slide frame (disclosed in JP Patent Publication (Kokai) No. 57-63452 A (1982)), to produce slide 1 for reflected light quantity measurement.

### (2) Method for producing multilayer analysis element for reflected light quantity measurement using a woven fabric as a spreading layer

A reagent layer was produced in a manner similar to that in (1) the method for producing a multilayer analysis element for reflected light quantity measurement using a polysulfone film as a spreading layer. Water was supplied in an amount of approximately 30 g/m² onto the whole surface of the above reagent layer, so as to impregnate the layer with water. Tricot woven fabric (hereinafter referred to as woven fabric) produced through 36 gauge-knitting of polyethylene terephthalate spun yarn having a fineness of 50 deniers was laminated onto the layer using slight pressure. The laminated product was dried, so as to cause the woven fabric to adhere to the layer. As described above, a multilayer analysis element for reflected light quantity measurement was produced using the woven fabric as a spreading layer.

The above multilayer analysis element was cut into a 12 mm×13 mm chip. The chip was set in a slide frame (disclosed in JP Patent Publication (Kokai) No. 57-63452 A (1982)), to produce slide 2 for reflected light quantity measurement.

### (3) Comparison of nonuniformities in reflected light quantity

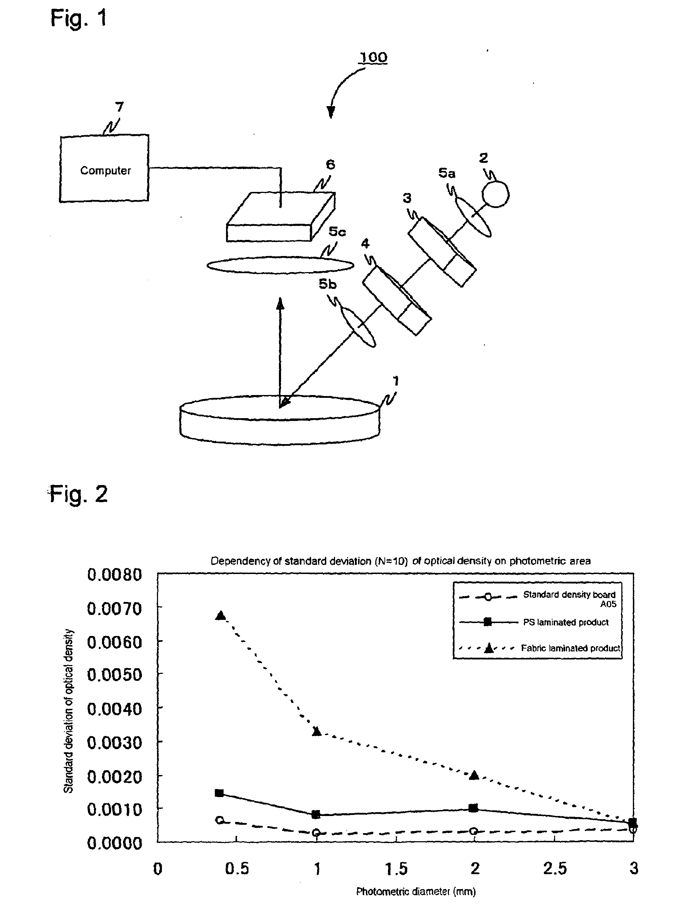

The above slides 1 and 2 for reflected light quantity measurement were subjected to measurement from the support side using a photometric system of the optical arrangement as shown in Fig. 1. Specifically, reflection photometry was performed with a photometric area of a diameter of 6 mm at 33 µm/pixel. The resulting coefficients of variation (CV(%)) of reflective ODs were compared. Table 9 shows the results.
Optical system: The optical system of an inverted stereoscopic microscope was used. Magnifications at the CCD light receiving part were x1 (10 µm/pixel at the CCD part) and ×0.33 (33 µm/pixel at the CCD part). Light source: Luminar Ace LA-150UX (produced by HAYASHI WATCH-WORKS CO., LTD.) Monochromated using an interference filter at 625 nm, 540 nm, or 505 nm Light attenuating filter: A glass filter ND-25 (produced by HOYA CORPORATION) and a filter prepared by (ourselves) making holes in a stainless plate were used.

CCD: 8-bit monochrome camera module XC-7500 (produced by SONY CORPORATION) was used.
Data processing: The thus obtained images were processed using an image processing apparatus LUZEX-SE (produced by NIRECO Corporation) for measurement.

As a means for calibration of reflection optical density, a standard density board (ceramic specification, produced by FUJIFILM Techno Products Co., Ltd.) was used. 6 types of the standard density board, A00 (reflection optical density to 0.05), A05 (reflection optical density to 0.5), A10 (reflection optical density to 1.0), A15 (reflection optical density to 1.5), A20 (reflection optical density to 2.0), and A30 (reflection optical density to 3.0) were used.

**Table 9. Comparison of coefficients of variation (CV(%)) of reflective ODs when photometric area with a diameter of 6 mm was subjected to reflection photometry at 33 µm/pixel**

| | Dry slide 1 for reflected light quantity measurement | Dry slide 2 for reflected light quantity measurement |
|---|---|---|
| Average reflective OD | 0.296 | 0.391 |
| Standard deviation(SD) | 0.010 | 0.097 |
| Coefficient of variation (CV(%)) | 3.4% | 25% |
| Maximum value | 0.333 | 0.771 |
| Minimum value | 0.260 | 0.275 |

It was confirmed that whereas the CV(%) of the reflective OD in the case of the slide 2 for reflected light quantity measurement using the woven fabric as a spreading layer was very high due to the network, the CV(%) of the reflective OD in the case of the slide 1 for reflected light quantity measurement using the PS film as a spreading layer was extremely low.

### (4) Comparison of dispersions measured with reduced photometric areas

The above slides 1 and 2 for reflected light quantity measurement were subjected to reflection photometry using a photometric system (10 µm/pixel) of the optical arrangement shown in Fig. 1. Reflection photometry was performed from the support side with repeated taking-out and putting-in (n=10) for photometric areas with diameters ranging from 3 mm to 0.4 mm. Thus, standard deviations (SDs) for these photometric areas were compared. As a control for comparison, similar measurement was performed for standard density board A05. Table 10 and Fig. 2 show the results.

**Table 10. Relationship between standard deviation (SD) of reflective OD and photometric area**

| Photometric diameter (mm) | 0.4 | 1 | 2 | 3 |
|---|---|---|---|---|
| Dry slide 1 for reflected light quantity measurement | 0.00142 | 0.00079 | 0.00097 | 0.00056 |
| Dry slide 2 for reflected light quantity measurement | 0.00673 | 0.00327 | 0.00200 | 0.00053 |
| Standard density board A05 | 0.00061 | 0.00024 | 0.00029 | 0.00034 |

The slide 2 for reflected light quantity measurement using the woven fabric as a spreading layer showed higher standard deviations of the reflective ODs when photometric areas were reduced. On the other hand, the slide 1 for reflected light quantity measurement using the PS film as a spreading layer showed small fluctuations in standard deviation. The use of the slide 1 enables measurement with high accuracy when a photometric area is reduced due to an extremely smaller amount of a liquid sample.

### Industrial Applicability

In the multilayer analysis element of the present invention, a non-fibrous porous film having a bending rupture strength of 20 gram-weight or more and a tensile percentage of 2% or less when the film is stretched with a tensile force of 50 gram-weight is used as a spreading layer. With the use of such film, an effect of not causing a broken spreading layer (during the step of adhering a spreading layer to a lower layer) or the like upon production can be obtained. Furthermore, an effect of not causing any changes in void volume because of low tensile percentage can be obtained. Accordingly, a multilayer analysis element can be provided, which results in small difference between lots, small difference within a lot, and high measurement accuracy, can be miniaturized, and enables stable production.

## Claims

1. A multilayer analysis element for liquid sample analysis wherein at least one functional layer and at least one porous liquid sample spreading layer of non-fibrous porous film are integrally laminated in this order on one side of a water-impermeable planar support, and the non-fibrous porous film has a bending rupture strength of 20 gram-weight or more and a tensile percentage of 2% or less when the film is stretched with a tensile force of 50 gram-weight, wherein said non-fibrous porous film of an organic polymer is polyethersulfone, polysulfone, or a mixture thereof.

2. The multilayer analysis element for liquid sample analysis according to claim 1, wherein said porous film is an asymmetric porous film with an asymmetry ratio of 2.0 or more.

3. The multilayer analysis element for liquid sample analysis according to claim 1, wherein said porous film is a symmetric porous film with an asymmetry ratio of less than 2.0.

## Patentansprüche

1. Mehrschichtiges Analyseelement für eine Flüssigprobenanalyse, in dem wenigstens eine funktionelle Schicht und wenigstens eine poröse Flüssigproben-Ausspreitungsschicht aus nicht-fasrigem porösem Film integral in dieser Reihenfolge auf eine Seite eines wasserundurchlässigen planaren Trägers laminiert sind, und wobei der nicht-fasrige poröse Film eine Biegereißfestigkeit von 20 Gramm Gewicht oder mehr und einen Zugprozentwert von 2% oder weniger hat, wenn der Film mit einer Zugkraft von 50 Gramm Gewicht gezogen wird, wobei der nicht-fasrige poröse Film eines organischen Polymers Polyethersulfon, Polysulfon oder ein Gemisch davon ist.

2. Mehrschichtiges Analyseelement zur Flüssigprobenanalyse gemäß Anspruch 1, wobei der poröse Film ein asymmetrischer poröser Film mit einem Asymmetrieverhältnis von 2,0 oder mehr ist.

3. Mehrschichtiges Analyseelement zur Flüssigprobenanalyse gemäß Anspruch 1, wobei der poröse Film ein symmetrischer poröser Film mit einem Asymmetrieverhältnis von kleiner als 2,0 ist.

## Revendications

1. Elément analytique multicouche pour l'analyse d'un échantillon de liquide, dans lequel au moins une couche fonctionnelle et au moins une couche de diffusion d'échantillon de liquide poreuse réalisée avec un film poreux non fibreux sont stratifiées de manière solidaire dans cet ordre d'un côté d'un support plan imperméable à l'eau, et le film poreux non fibreux a une résistance de rupture à la flexion de 20 grammes en poids ou plus et un pourcentage de traction de 2 % ou moins lorsque le film est étiré avec une force de traction de 50 grammes en poids, dans lequel ledit film poreux non fibreux d'un polymère organique est du polyéther sulfone, du polysulfone ou leur mélange.

2. Elément analytique multicouche pour l'analyse d'un échantillon de liquide selon la revendication 1, dans lequel ledit film poreux est un film poreux asymétrique avec un rapport d'asymétrie de 2,0 ou plus.

3. Elément analytique multicouche pour l'analyse d'un échantillon de liquide selon la revendication 1, dans lequel ledit film poreux est un film poreux symétrique avec un rapport d'asymétrie inférieur à 2,0.
